Europäisches Patentamt

(19) European Patent Office

Office européen des brevets

(11) Publication number: **0 127 983**
A2

(12) **EUROPEAN PATENT APPLICATION**

(21) Application number: **84303494.3**

(22) Date of filing: **23.05.84**

(51) Int. Cl.³: **H 01 J 35/14**
**A 61 B 6/02**

(30) Priority: **01.06.83 US 500136**

(43) Date of publication of application:
**12.12.84 Bulletin 84/50**

(84) Designated Contracting States:
**DE FR GB IT NL SE**

(71) Applicant: **IMATRON INC.**
**389 Oyster Point Boulevard**
**South San Francisco California 94080(US)**

(72) Inventor: **Rand, Roy Edward**
**1028 McGregor Way**
**Palo Alto California 94306(US)**

(74) Representative: **Cross, Rupert Edward Blount et al,**
**BOULT, WADE & TENNANT 27 Furnival Street**
**London EC4A 1PQ(GB)**

(54) Scanning electron beam computed tomography scanner.

(57) A scanning electron beam computed tomography scanner includes means defining a vacuum chamber (16), means (21, 22) for producing a space-charge limited electron beam (24) at one location in the chamber (16) and for directing it to a second location therein, and an elongate target (26) of the type which produces X-rays as a result of the impingement thereon by the electron beam (24). The scanner also includes an arrangement (42) at the second location for causing the beam (24) to scan along the length of the target (26) so as to impinge on the latter at all points along its length in order to cause X-rays to be produced by and emanate from the target (26). This arrangement includes means, specifically differential focal strength electron beam optics, for reducing the convergence of the electron beam (24) in the radial plane, at any instant, while maintaining the position of the waist of the beam (24) in the scan plane at the location of the target (26), thus reducing the mutual repulsion of the electrons in the beam and decreasing the azimuthal width of the electron beam spot. This also decreases the azimuthal width of X-ray beam effective focal spot and thereby improves the resolution of the scanner. The increased radial size of the electron beam spot is compensated by orienting the target (26) relative to the electron beam (24) in a specific way so that the axial width of the X-ray effective focal spot is independently controlled.

FIG.—1

SCANNING ELECTRON BEAM
COMPUTED TOMOGRAPHY SCANNER

The present invention relates generally to a technique for controlling a scanning electron beam which produces X-rays in a computed tomography X-ray transmission scanner and more particularly to the use of differential focal strength beam optics to improve overall resolution of the scanner.

There are a number of different types of X-ray transmission scanning systems described in the literature, including that described in U.S. Patent No. 4,352,021 (Boyd et al). In this latter system an electron beam is produced inside a highly evacuated chamber by an electron gun. The beam expands from its point of origin, because of the mutual electrostatic repulsion of the electrons in the beam. Where the beam is sufficiently large, it passes through a magnetic lens (solenoid) and a dipole deflecting magnet which bends the beam (in the bend plane) and, at the same time, scans it azimuthally along a tungsten target located at the far end of a conical vacuum chamber. For purposes of clarity, the following is to be understood. The bend plane of the electron beam is the plane containing the axes of the incident and deflected beams and the scanner axis. The bend plane is also referred to as the radial plane of the scanner. The transverse plane of the electron beam is perpendicular to the bend plane and contains the axis of the deflected beam. The transverse plane is also referred to as the scan plane.

The position of these planes changes continuously as the deflected electron beam rotates in the scan tube. The radial direction is a direction in the radial plane perpendicular to the deflected beam axis. The azimuthal direction is perpendicular to the radial direction and to the scanner axis. The axial direction is parallel to the scanner axis.

The solenoid referred to above serves to focus the beam to a small spot on the target which results in the production of an X-ray beam having an effective focal spot size which depends on the size of the electron beam spot. Throughout the path of the electron beam, the self forces of the beam are dominated by its space charge, i.e. the electrons mutually repel each other. This repulsion limits the minimum beam spot size (and therefore the effective focal spot size of the X-ray beam), which in the case of unit beam optical magnification, and cylindrical symmetry with respect to the beam axis is equal to the size of the initial electron beam waist at the electron gun.

The foregoing ignores the fact that the focusing properties of the dipole are not cylindrically symmetric. Generally the dipole will have a greater focal strength in the transverse plane than in the bend plane. This causes the principal points of the solenoid/dipole combination to be closer to the target in the transverse plane than in the bend plane. Consequently, for purely beam optical reasons (i.e. for an electron beam with negligible space charge), the azimuthal (transverse) electron beam spot size is less than the radial (bend plane) beam spot size. This is a desirable feature from the point of view of the overall spatial resolution of the scanner since the X-ray effective focal spot size in the azimuthal direction (scanning direction) is small while the larger size in the axial direction, which corresponds to the radial width of the electron beam (normal to the scanning direction), can be compensated by adjusting the target angle, as will be seen herein. In this regard it should be noted that the azimuthal beam spot widths are the same for X-rays and electrons.

The desirable focusing feature just mentioned relies on the relative displacement of the principal points in two planes. As will be seen hereinafter, for a space-charge limited beam, there is a similar but more significant benefit to be gained from employing the preferred embodiment of the present invention which is directed to the use of a differential focal strength beam optic arrangement and which does not depend on a displacement of the principal points in the two planes. In fact the effect can be illustrated using a simple thin lens approximation. Differential focal strength under this condition is of no advantage beam optically, i.e. for very low current beams with negligible space charge.

The formation of a generally non-cylindrically symmetric beam (by a focal strength difference) results in a reduction in the self repulsion of the beam due to its own space charge. Advantage may be taken of this effect, with high beam currents, to reduce considerably the azimuthal beam spot size, compared to the cylindrical beam case, while the radial beam spot width increases only moderately and need be no bigger than the low current beam spot width with the same focal strength difference. The target angle can of course be adjusted to compensate for this radial increase if necessary. In principle, by choosing an appropriate focal strength difference and target angle, the X-ray effective focal spot dimensions at high beam currents can be reduced substantially which improves the scanner's overall resolution.

The advantage to be gained from the differential focal strength is calculated herein and has been confirmed by experiment.

It should be noted that for a space-charge limited electron beam a smaller beam width in the azimuthal direction can only be achieved with a greater focal strength in the transverse plane than in the bend plane. In general, the beam must not reach a waist in the bend plane before it strikes the target.  The reduction in azimuthal beam size is not determined uniquely by the initial slope of the beam envelope in the transverse plane, but depends on the beam's behavior in both planes.

An essential component of the scanner when used in this mode is a set of magnetic quadrupole coils which are installed inside the dipole magnets.  These coils are used to optimize the difference in focal strengths of the composite magnetic lens (solenoid, dipole, quadrupole).  Also, since the dipole field is rotating, the quadrupole currents must be scanned in synchronism with the dipole currents but at twice the frequency.

The preferred embodiment of the present invention, as briefly referred to above, will be described in more detail hereinafter as will be the present invention generally. Both are directed to a scanning electron beam computed tomography scanner which comprises means defining a vacuum chamber, means for producing a space-charge limited electron beam at one location in the chamber and for directing the beam to a second location therein, and an elongated target (also located within the chamber) of the type which produces X-rays as a result of the impingement thereon by the electron beam.  The scanner also includes an arrangement for causing the electron beam to scan along the length of the target so as to impinge the latter at all points along its length so as to cause X-rays to be produced by and emanate from the target.  In accordance with the present invention, this arrangement includes means for causing the electron beam to converge less in one plane than in an orthogonal plane, thus producing a cross-section of the beam at its point of impingement with the target generally elliptical in configuration and fixed in size at all points along the length of the target.  In a preferred embodiment, the means for producing the differential convergence of the electron beam and for maintaining the beam's cross-section elliptical in configuration and fixed in size includes means for focusing the beam to a spot on the target, the focusing means specifically displaying a greater focal strength in the scan plane of the beam, at any instant along its scan path, than the focal strength in the radial plane at that instant. This is accomplished specifically by means of an assembly of dipole magnets containing a set of magnetic quadrupole coils.

The present invention and its preferred embodiment will be described in more detail hereinafter in conjunction with the drawings wherein:

FIGURE 1 diagrammatically illustrates the scanning electron beam computed tomography scanner designed in accordance with the present invention;

FIGURE 1A diagrammatically illustrates an X-ray producing target assembly forming part of the scanner of Figure 1;

FIGURES 2A and 2B diagrammatically illustrate how the scanner of Figure 1 directs an electron beam having a specific cross-sectional configuration onto the X-ray producing target of Figure 1A to produce X-rays.

FIGURES 2C, 2D and 2E diagrammatically illustrate how the differential focal strength beam optics of the scanner shown in Figure 1 produces less convergence of the electron beam in the radial plane than in the scan plane;

FIGURES 2F and 2G diagrammatically illustrate how a target and electron beam just referred to cooperate with one another to produce an X-ray beam effective focal spot with improved axial width;

FIGURE 3 is a graphic illustration of calculated beam envelopes (HWHM) for 100 keV electron beams from the lens to the scanner target in a scanner of the type illustrated in Figure 1 and specifically with regard to cylindrical symmetry and beam currents 0, 300, 600, 900 mA;

FIGURE 4 is a graphical illustration similar to the one shown in Figure 3 for elliptical beams generated by a lens strength difference $(1/f_y - 1/f_x)$ of $0.08m^{-1}$, the y (azimuthal) envelope being plotted upwards and the x (radial) envelope downwards;

FIGURE 5 is a graphical illustration similar to the one shown in Figure 4 for a lens strength difference of $0.16m^{-1}$;

FIGURE 6 diagrammatically illustrates calculated X-ray effective focal spots due to elliptical electron beams with the parameters set forth and with a reduction in axial dimension, due to the target angle, equal to 1/5; and

FIGURE 7 graphically illustrates experimentally measured beam envelopes for 100 keV, 350 mA electron beam with lens strength difference approximately equal to $0.16m^{-1}$.

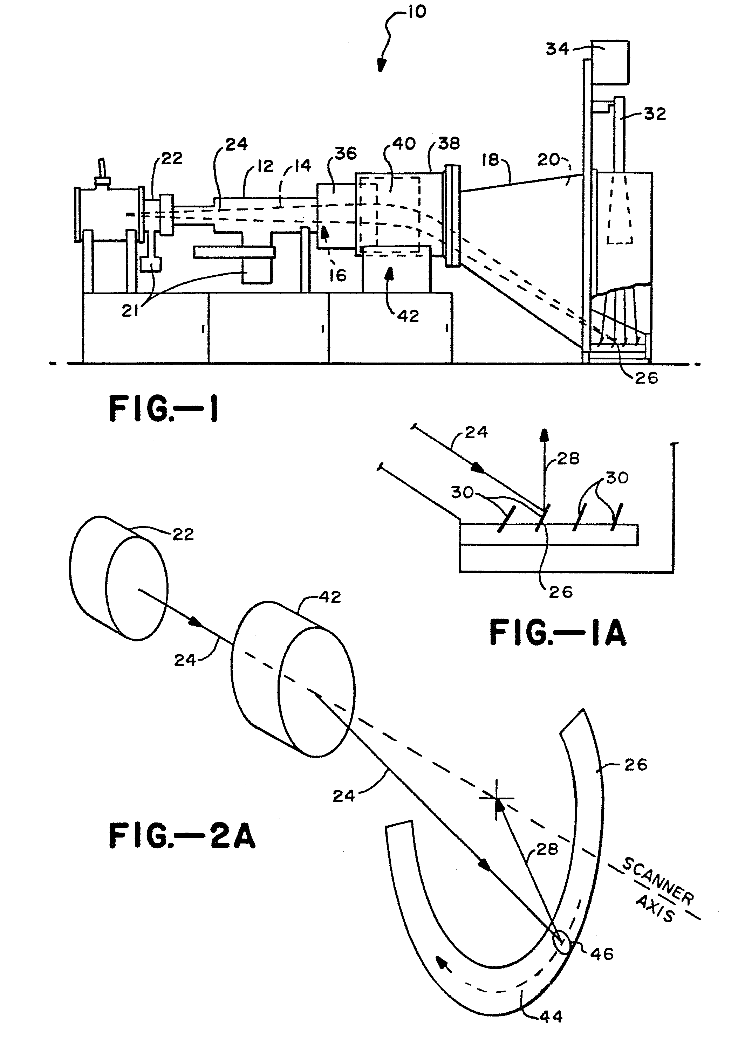

Turning now to the drawings, attention is first directed to Figure 1 which illustrates a scanning electron beam computed tomography scanner designed in accordance with the present invention and generally indicated by the reference numeral 10. With the exception of the present invention which will be discussed below, scanner 10 may be identical to the one described in previously recited Boyd et al patent 4,352,021 and/or the scanner disclosed in applicant's own pending application Serial No. 434,252, filed October 14, 1982 (hereinafter referred to as the Rand application). To this end, the scanner includes means generally indicated at 12 for defining one section 14 of a vacuum chamber 16, means generally indicated at 18 for defining a second section 20 of the same chamber, and a vacuum pump 21 acting on the chamber. Means including an electron gun 22 and its own vacuum pump 21 located at the rearwardmost end of chamber 16 serves to produce a space-charge limited electron beam 24 which is directed horizontally in a continuously expanding fashion through chamber section 14 to the forwardmost end of the latter where it is acted upon in the manner to be discussed hereinafter. The cross-section of electron beam 24 between electron gun 22 and the forwardmost end of chamber section 14 is circular in configuration.

As illustrated in Figure 1, means 18 defining chamber section 20 is configured to define a somewhat cone-shaped chamber section, at least to the extent that it tapers downward and outward from chamber section 14. At the forwardmost end of this chamber section, scanner 10 includes a set of targets of the type which produce X-rays 28 (see Figure 1A) as a result of the impingement thereon by electron beam 24. A typical target 26 is generally arcuate in form and extends around the inside of chamber section 20 at its forwardmost end. As best seen in Figure 1A, the targets form a series of stepped surfaces 30 which extend the entire length of the targets and which face the rearwardmost end of chamber section 20 in order to intercept and, as a result, be impinged by electron beam 24 in order to produce a resultant X-ray beam 28. The way in which electron beam 24 is directed onto target surfaces 30 forms part of the present invention and will be discussed hereinafter. For the moment, it suffices to say that surfaces 30 are angled relative to the incoming electron beam to reduce the axial width of the effective focal spot of the resultant X-ray beam. The X-ray beam 28 is directed upward through a patient (not shown) to one of a number of detectors 32. Overall operation of the scanner is controlled by computer processing means partially indicated at 34.

The components making up overall scanner 10, as described thus far, form part of the previously recited Boyd et al patent and/or applicant's pending patent application Serial No. 434,252 and therefore will not be described herein. Rather, reference is made to the Boyd et al patent and the Rand patent application. In this regard, it should be noted that scanner 10 may include other components (not disclosed herein) which do not form part of the present invention but which are necessary or desirable to operation of the overall scanner. For a discussion of these components, reference is again made to the Boyd et al patent and the Rand patent application.

In accordance with the present invention, scanner 10 includes a solenoid coil 36 and an assembly of dipole coils 38, the latter containing a set of magnetic quadrupole coils 40. While these three components, that is, the solenoid coil, assembly of dipole coils and the set of quadrupole coils, are individually known in the art, they are combined in accordance with the present invention to provide an overall arrangement 42 at the forwardmost end of chamber section 16 and the rearwardmost end of chamber section 20 for causing beam 24 to scan along the length of typical target 26 so as to impinge one of the stepped surfaces 30 at all points along its length in order to cause X-rays 28 to be produced by and emanated from the target. As will be seen hereinafter, this arrangement of components maintains the convergence of the electron beam 24 greater in the scan plane than in the radial plane and thus the cross-section of electron beam 24 at its point of impingement with the target is generally elliptical in configuration (rather than circular) and fixed in size at all points along the length of the target. Moreover, the orientation of the electron beam's cross-sectional configuration at its impingement point is fixed relative to the scan path of the beam. Specifically, in the embodiment illustrated, the major axis of the elliptical beam at its point of impingement is always normal to its scan path. As will also be seen hereinafter, this is accomplished by means of quadrupole coils 40 in combination with the dipole coils to focus the electron beam to a spot on the target utilizing differential focal strength optics. This, in turn, can be used to reduce the azimuthal size of the spot on the X-ray target over and above what is otherwise possible with a circular beam spot when the beam is space-charge limited as discussed previously, and thereby improve the overall resolution of scanner 10.

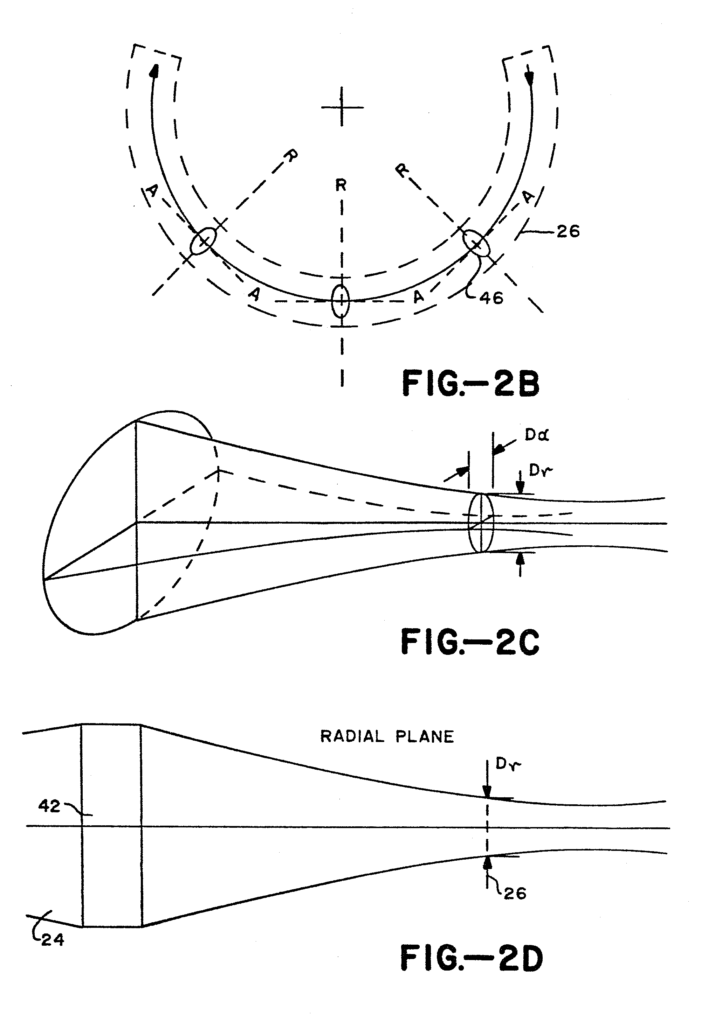

The present invention is best exemplified in Figures 2A-2G. First referring to Figure 2A, electron beam 24 is shown diagrammatically in its scanning mode relative to arcuate target 26. In this diagrammatic illustration, the arcuate scan path of the electron beam is indicated at 44 and corresponds to one arcuate length of the target. This scan path at any given point thereon is defined by the intersection of perpendicular A (azimuthal) and R (radial) axes, as best illustrated in Figure 2B. The azimuthal axis is in the direction of movement of the beam and the radial axis is perpendicular thereto. Overall arrangement 42 focuses electron beam 24 to a spot 46 on the surface 30 of target 26, as best shown in Figure 2B. In accordance with the present invention, this spot is generally elliptical in configuration and fixed in size at all points along the length of target 26 and, just as important, the major axis is always normal to the scan path, i.e., in the radial direction, while the minor axis is always in the azimuthal direction. As indicated above, this is the result of differential focal strength optics, as best diagrammatically illustrated in Figures 2C, 2D and 2E.

As seen in Figures 2C, 2D and 2E, arrangement 42 intercepts expanding electron beam 24 (indicated by dotted lines in Figures 2D and 2E) and "focuses" it onto target 26 (also indicated by dotted lines in Figures 2D and 2E) with different focal strengths in the azimuthal and radial planes. As seen in Figure 2D, the convergence of the focused beam in the radial plane is substantially less than the convergence of the beam in the scan plane. As a result, the cross-section of this beam at the target (and actually at all points on the length of the beam between arrangement 42 and target 26) is elliptical in shape, as best seen in Figure 2C. Note that the major axis of this ellipse lies in the radial direction while the minor axis lies in the azimuthal direction. As stated previously, as beam 24 is scanned along the length of target 26, the major axis is always maintained in the radial direction, i.e. perpendicular to the scan path, while the minor axis is always maintained tangential to the scan path.

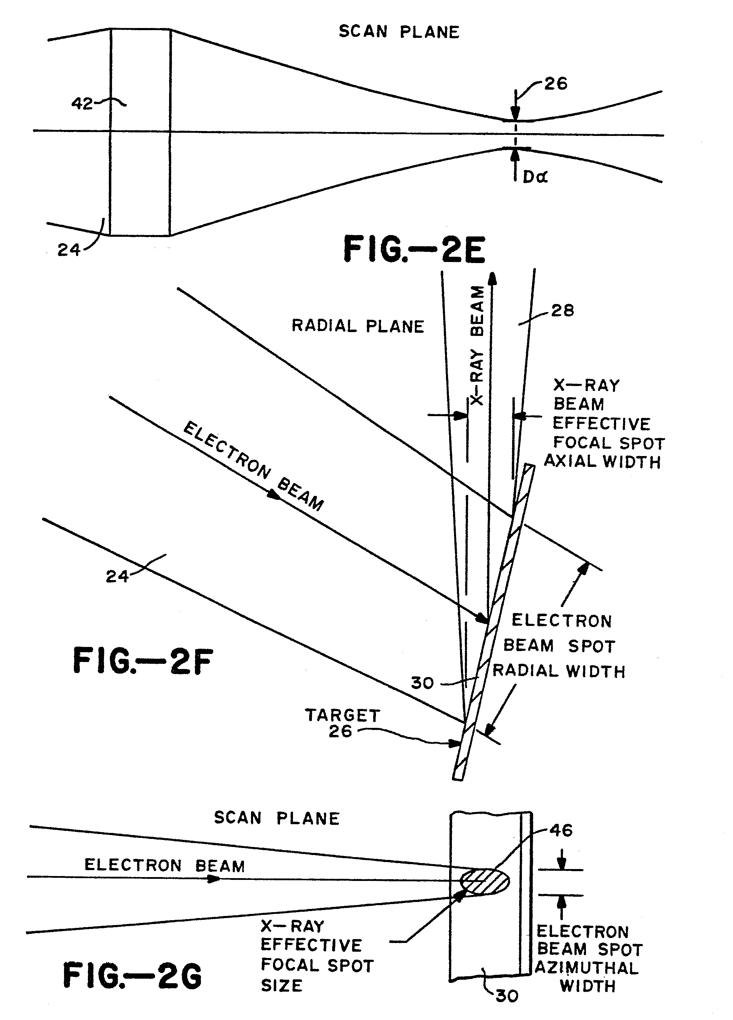

The reason for providing differential focal strength beam optics is to reduce the convergence of the electron beam 24 and so increase its width in the radial plane, while still maintaining the position of the waist of the beam in the scan plane at the location of the target 26. This reduces the mutual repulsion of the electrons in the space-charge limited beam, compared to the repulsion in a beam with circular cross-section, and so the size of the beam waist in the scan plane, which is the azimuthal width, $D_a$ of the electron beam spot on the target, is decreased. Since $D_a$ is also the azimuthal width of the X-ray beam effective focal spot, the spatial resolution of the scanner is improved. When the azimuthal width of the electron beam spot is decreased in this way, its radial width, $D_r$ is increased. Thus the electron beam spot becomes elliptical in form. The orientation of the ellipse on the target as well as the difference in focal strength between the two planes are controlled by the quadrupole coils within the dipole. The increased radial width of the electron beam spot is of no concern since the axial width of the X-ray beam effective focal spot does not depend on this radial width alone, but can be reduced by modifying the angle of the surface 30 of the target. This is best exemplified in Figures 2F and 2G which diagrammatically illustrate the interrelation between the surface 30 of target 26 and the electron and X-ray beams 24 and 28.

Figure 2F specifically illustrates how the electron beam impinges target surface 30 in the radial plane. The X-ray beam 28 resulting from the impingement of the electron beam with the target is shown in the same plane. Note that, because of the angular relationship between the incoming electron beam and the target surface, the axial width of the X-ray beam effective focal spot is substantially smaller than the radial width of the electron beam spot. It should be apparent from Figure 2F that the angular relationship between the incoming beam and the target surface can be varied so as to vary the relationship between the electron beam spot radial width and the X-ray beam effective focal spot axial width in the same plane. However, the important point to note is that the X-ray beam effective focal spot axial width in the radial plane can be made to be narrower than the electron beam spot in the same plane merely by providing the appropriate angular relationship between the electron beam and target surface. This has the same effect with respect to the X-ray beam as reducing the size of the electron beam in the same plane without having to actually do so. On the other hand, given the same angular relationship between the electron beam and target surface, it should be apparent from Figure 2G that the azimuthal width of the X-ray beam effective focal spot in the scan plane is equal to the width of the electron beam spot in the same plane, regardless of the actual angle between the electron beam and the target surface. Therefore, in order to reduce the size of the X-ray beam effective focal spot in the scan plane it is necessary to reduce the size of the electron beam spot in the scan plane. As indicated above, the electron beam spot can be reduced in the scan plane at the expense of the beam size in the radial plane by providing the previously described differential focal strength beam optics. By orienting the resulting elliptical beam spot such that its major axis lies in the radial plane, the additional width in this plane can be compensated for by the angular relationship between the electron beam and the target. At the same time, the minor axis which lies in the scan plane and which cannot be compensated for by the angular relationship just mentioned is made as small as is required. This results in an X-ray beam effective focal spot having an overall cross-sectional configuration which is smaller than would otherwise be possible by using a cylindrically symmetric space-charge limited electron beam and thereby improves the overall resolution of scanner 10.

In order to more fully understand the present invention, attention is now directed to the theory of elliptical space-charge limited electron beam behavior. The equations which describe the beam envelope of an elliptical cross-section electron beam with uniform current density, under the influence of self forces (electrostatic and magnetic) are

$$\left. \begin{array}{l} \dfrac{d^2x}{dz^2} = \dfrac{S}{x+y} + \dfrac{\epsilon^2}{x^3} \\[4mm] \dfrac{d^2y}{dz^2} = \dfrac{S}{x+y} + \dfrac{\epsilon^2}{y^3} \end{array} \right\} \quad (1)$$

where     x is the transverse dimension in the radial plane,

y is the transverse dimension in the scan plane,

z   is in the direction of electron motion,

S   describes the self forces,

and   $\epsilon$   is the beam emittance.

The parameter S is given by

$$S = \sqrt{2m} \; \eta_o KI/I_{SAT}$$

where   m is the electron rest mass (in volts),

$\eta_o = 30\,\Omega$   is the resistance of free space,

K is the perveance of the electron gun,

and $I_{SAT}$ is the saturated beam current given by:

$$I_{SAT} = K \left[ T \, (1+T/2m) \right]^{3/2}$$

where T is the kinetic energy of a beam electron (in volts).

The expanding section of the beam in the scanner is cylindrically symmetric. In this case, eqns. (1) reduce to:

$$\frac{d^2 r}{dz^2} = \frac{s}{2r} + \frac{\epsilon^2}{r^3} \tag{2}$$

where      r is the beam radius.

The solution of this equation has been discussed elsewhere. In this case it is used primarily to calculate the beam envelope radius at the magnetic lens for a given beam waist radius at the electron gun.

The solution of eqns.(1) for the converging part of the beam requires numerical analysis. In this paper a solution in terms of Taylor series has been used, with terms up to and including fourth derivatives. The solution is:

$$\left. \begin{array}{l} x = x_0 + h\left(\dfrac{dx}{dz}\right)_0 + \dfrac{h^2}{2}\left(\dfrac{d^2 x}{dz^2}\right)_0 + \dfrac{h^3}{6}\left(\dfrac{d^3 x}{dz^3}\right)_0 + \dfrac{h^4}{24}\left(\dfrac{d^4 x}{dz^4}\right)_0 + \cdots \\[4mm] \dfrac{dx}{dz} = \left(\dfrac{dx}{dz}\right)_0 + h\left(\dfrac{d^2 x}{dz^2}\right)_0 + \dfrac{h^2}{2}\left(\dfrac{d^3 x}{dz^3}\right)_0 + \dfrac{h^3}{6}\left(\dfrac{d^4 x}{dz^4}\right)_0 + \cdots \end{array} \right\} \tag{3}$$

where: $z = z_0 + h$

$$\frac{d^3 x}{dz^3} = \frac{-s}{(x+y)^2}\left[\frac{dx}{dz} + \frac{dy}{dz}\right] - \frac{3\epsilon^2}{x^4}\left(\frac{dx}{dz}\right)$$

$$\frac{d^4 x}{dz^4} = \frac{2s}{(x+y)^3}\left[\left(\frac{dx}{dz} + \frac{dy}{dz}\right)^2 - s\right] - \frac{s\epsilon^2}{(x+y)^2}\left[\frac{1}{x^3} + \frac{1}{y^3}\right]$$

$$+ \frac{12\epsilon^2}{x^5}\left(\frac{dx}{dz}\right)^2 - \frac{3\epsilon^2}{x^4}\left(\frac{d^2 x}{dz^2}\right)$$

where the suffix "o" refers to the initial conditions for each increment in z.

Similar equations may be written for the derivatives of y.

To illustrate the benefits of differential focal strength beam optics, beam envelope calculations have been performed using equations (1) and a model which describes approximately the prototype scanning electron beam X-ray tube.

The magnetic lens is described by the thin lens approximation. Fixed parameters used are: electron beam kinetic energy= 100keV; beam emittance= 11.1πmm mr; gun to lens distance= 200cm; lens to target distance= 200cm; beam radius (half width half maximum) at lens (both planes)= 5.0cm. For each set of conditions, the initial slope of the beam envelope in the scan (y) plane is chosen to produce a transverse beam waist at the target. (This is not necessarily the condition for the smallest beam width at the target, but is generally close to that optimum.) Variable parameters used are: scan plane lens strength minus radial plane lens strength= 0 (cylindrical), $0.08m^{-1}$ and $0.16m^{-1}$; beam current= 0, 300mA, 600mA and 900mA. (For the half width half maximum beam envelope, the currents inside the envelope are three quarters of these values. The corresponding values of S are 0, $1.875 \times 10^{-4}$, $3.75 \times 10^{-4}$, $5.625 \times 10^{-4}$.)

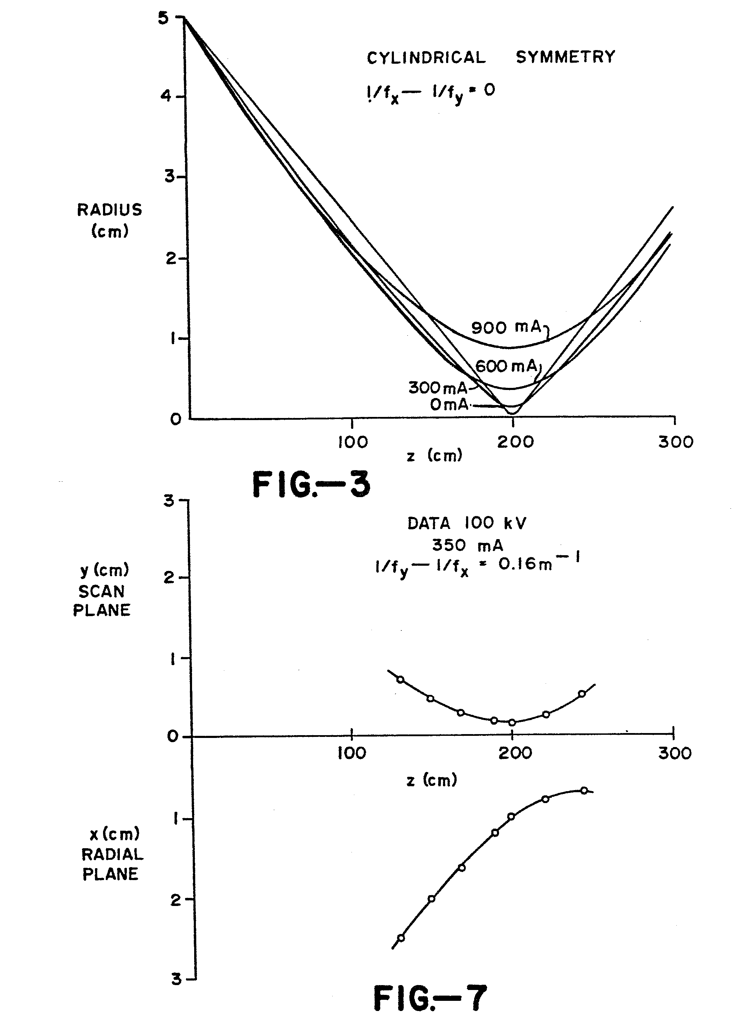

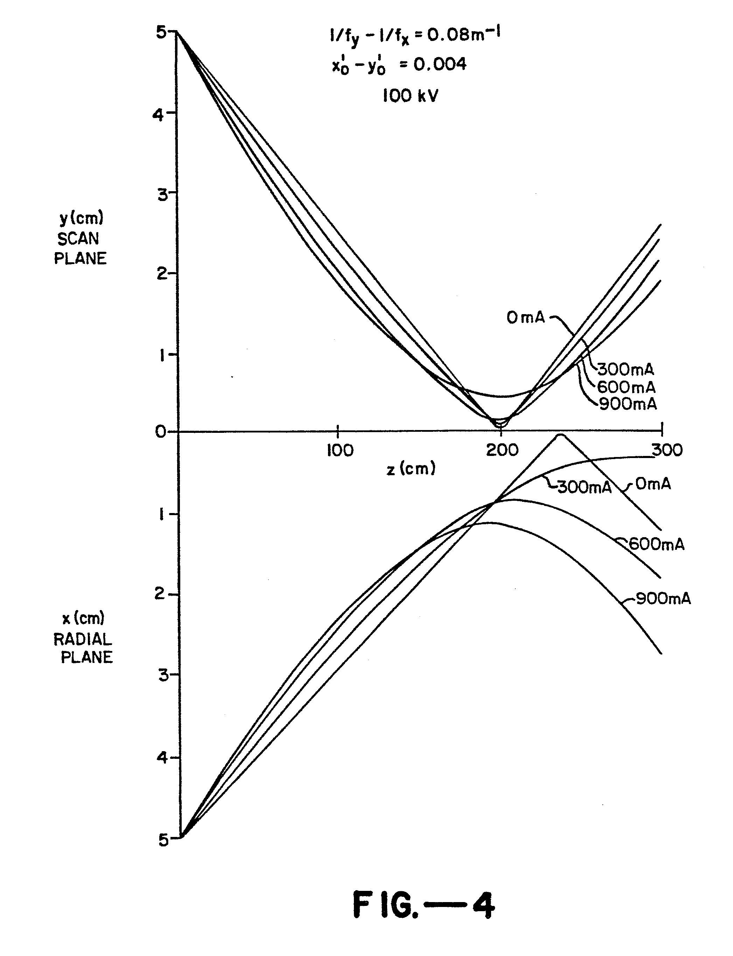

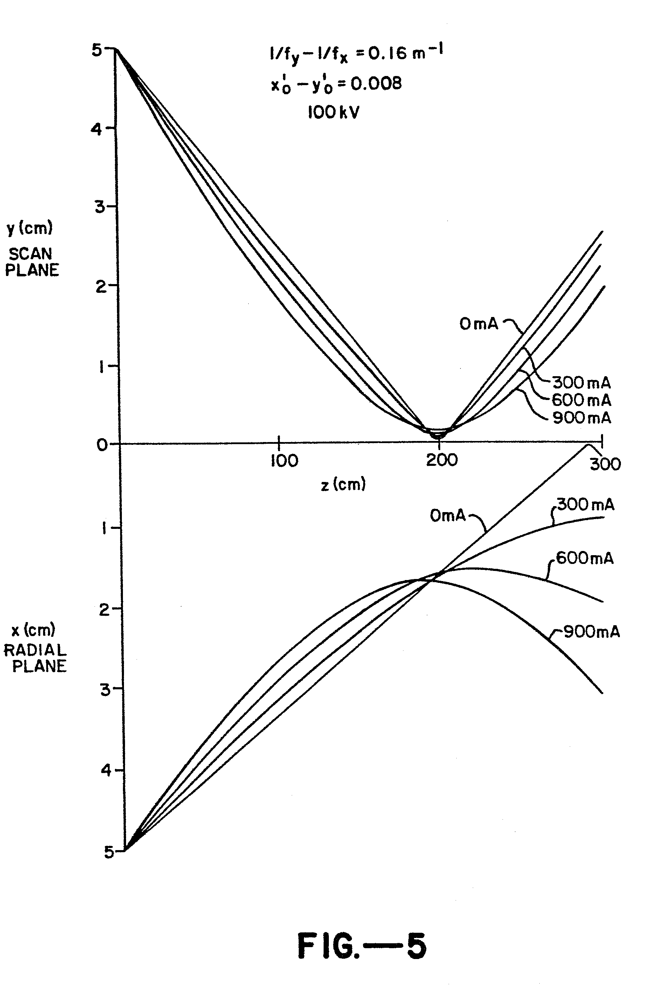

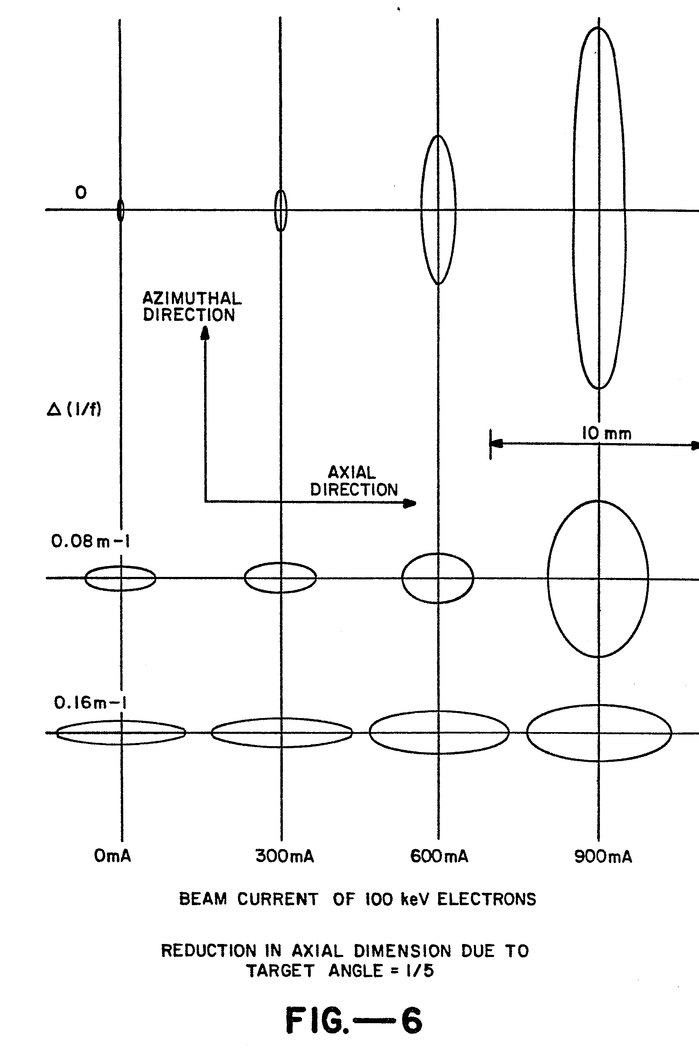

The results of the calculations are shown by the x, y beam envelope profiles in Figures 3, 4 and 5. These clearly show the reduction in the transverse width of the beam spot as the focal strength difference is increased. Assuming a target angle which reduces the axial width of the X-ray effective focal spot to one fifth of the radial width of the electron beam spot, the forms of the X-ray effective focal spot are plotted in Figure 6. It can be seen that for each beam current, the azimuthal width of the X-ray beam effective focal spot, which determines scanner resolution, can be reduced as required by increasing the difference in focal strength, while the axial width of the X-ray beam effective focal spot grows only slowly. For instance, for a 900mA electron beam, the azimuthal diameter of the X-ray beam effective focal spot, produced by increasing the focal strength difference from zero to $0.16m^{-1}$, is reduced by a factor of 5.3, while the axial diameter increases by only a factor of 2.0. This increase can be further reduced by modifying the target angle.

Beam profiles actually measured in the prototype scanner for 350mA of 100keV electrons are shown in Figure 7. These measurements show the same trends as the calculations but do not correspond exactly since the actual beam cross-section at the lens is not precisely circular.

In summarizing the foregoing, it has been shown that differential focal strength electron beam optics may be used in a scanning electron beam computed tomography scanner to reduce the X-ray beam azimuthal effective focal spot width with only a moderate increase in the axial beam spot width. This effect is a result of the properties of a space-charge limited elliptical electron beam and can only be achieved if the beam is focused more strongly in the scan plane than in the radial plane so that in general the waist in the scan plane occurs first or very close to the radial plane waist. Although the deflecting dipole in the scanner naturally focuses the electron beam in the required manner, it is necessary to use auxiliary magnetic quadrupole coils in conjunction with the dipoles and solenoid to optimize the focal strength difference and to maintain this difference constant as the beam is scanned along the target.

In further summarizing the foregoing, it should be noted that the overall scanner was described with respect to a specific positional relationship between various planes, particularly the radial plane and the scan plane. Obviously, this positional relationship could be varied without imparting from the present invention which lies in the ability to reduce the cross-section of the X-ray beam effective focal spot by means of differential focal strength electron beam optics in conjunction with a physical angular relationship between the electron beam and the target. In other words, scanner 10 could be designed to provide a different positional relationship between its various components without departing from the present invention.

CLAIMS:

1.     A scanning electron beam computed tomography scanner, comprising means defining a vacuum chamber; means for producing a space-charge limited electron beam at one location in said chamber and for directing it to a second location therein; an elongated target of the type which produces X-rays as a result of the impingement thereon by said electron beam; and an arrangement at said second location for causing said beam to scan along the length of said target so as to impinge on the latter at all points along its length in order to cause X-rays to be produced by and emanate from said target, said arrangement including means for causing the electron beam to converge to a spot on said target, said beam being caused to converge less in one plane than in an orthogonal plane, in order to provide a reduction in the mutual repulsion of the electrons in the beam and to cause the beam to have a smaller dimension at its point of impingement with said target, in the direction of the plane of greater convergence, whereby the resulting beam spot is generally elliptical in configuration and fixed in size at all points along the length of said target.

2.     A scanner according to Claim 1 wherein said means for causing the electron beam to converge less in one plane than in an orthogonal plane and for keeping the beam spot elliptical and fixed in size, includes means for maintaining the orientation of the beam's cross-sectional configuration at said impingement point fixed relative to the scan path of said beam.

3.     A scanner according to Claim 2 wherein the plane of least convergence is always normal to said scan path, resulting in the major axis of the elliptical cross-sectional configuration of said beam at said impingement point being always normal to said scan path.

4. A scanner according to Claim 2 or Claim 3, wherein the entire length of said X-ray target is oriented relative to said electron beam such that the cross-section of said X-ray beam's effective focal spot as it emanates from said target has a first axis equal in length to and dependent on the minor axis of the elliptical electron beam spot and a second axis less than and only partially dependent on the major axis of said beam spot.

5. A scanner according to any preceding claim, wherein said means for causing the electron beam to converge less in one plane than in an orthogonal plane and for maintaining the cross-section of said beam spot elliptical in configuration and fixed in size includes means for focusing said beam to a spot on said target, said focusing means displaying a greater focal strength in the plane of movement of said beam, at any instant along its scan path, than the focal strength in a plane which is normal to said last-mentioned plane and which also contains the axis of the electron beam at that instant.

6. A scanner according to Claim 5, wherein said focusing means includes an assembly of dipole magnets containing a set of magnetic quadrupole coils.

7. A scanner according to any preceding claim, wherein said target is arcuate in length.

8. A scanning electron beam computed tomography scanner, comprising means defining a vacuum chamber; means for producing an electron beam at one location in said chamber and for directing it to a second location therein; an elongate target of the type which produces X-rays as a result of the impingement thereon by said electron beam; and an arrangement at said second location for causing said beam to scan along the length of said target so as to impinge on the latter at all points along its length in order to cause X-rays to be produced by and emanate from said target, said arrangement including means at said second location for focusing said beam to a spot on said target, said focusing means displaying a greater focal strength in the plane of movement of said beam, at any instant along its scan path, than the focal strength in a plane which is both normal to said plane of movement and also contains the axis of the electron beam at that instant.

9. A scanner according to Claim 8, wherein said focusing means includes an assembly of dipole magnets containing a set of magnetic quadrupole coils.

10. A scanner according to Claim 8 or Claim 9, wherein said target is arcuate in length such that the scanning surface of movement of said electron beam is conical in configuration.

11. A scanning electron beam computed tomography scanner, comprising means defining a vacuum chamber; means for producing an electron beam having a generally circular cross-section at a first location within said chamber; means for directing said beam in a continuously expanding manner and along a horizontal axis from said first location to a second location in said chamber; an elongate target of the type which produces X-rays as a result of impingement thereon by said electron beam, said target being arcuate in length and being positioned within said chamber in confronting relationship with and partially below said second location; and an arrangement at said second location for redirecting said beam from said second location to said target so as to cause said beam to scan along the length of said target in order to impinge the target at all points along its length and thereby cause X-rays to be produced by and emanate from said target, said arrangement including means for focusing said beam from said second location to a spot on said target, said focusing means displaying a greater focal strength in the plane of movement of said beam, at any instant along its scan path, than the focal strength in a plane which is normal to said plane of movement and which also contains the axis of the electron beam at that instant, whereby the spot is elliptical in shape with its major axis always normal to the path of movement of the electron beam as the latter scans the length of said target.

12. A scanner according to Claim 11 wherein said target is oriented relative to said electron beam such that the cross-section of said X-ray beam effective focal spot as it emanates from said target has a first axis equal in length to and dependent on the minor axis of the electron beam cross-section and a second axis less than and only partially dependent on the major axis of said electron beam cross-section.

13. A method of producing X-rays in a scanning electron beam computed tomography scanner, comprising the steps of defining a vacuum chamber; producing an electron beam at one location in said chamber and directing it to a second location therein; providing an elongate  target of the type which produces X-rays as a result of the impingement thereon by said electron beam at a third location in said chamber; and, at said second location, causing said beam to scan along the length of said target so as to impinge on the latter at all points along its length in order to cause X-rays to be produced by and emanate from said target, said scanning step including the step of focusing said beam from said second location to a spot on said target, said focusing step causing said electron beam to converge more strongly in the plane of movement of said beam, at any instant along its scan path, than in a plane which is normal to said plane of movement and which also contains the axis of the electron beam at that instant.

0127983

FIG.—1

FIG.—1A

FIG.—2A

0127983

FIG.—2B

FIG.—2C

RADIAL PLANE

FIG.—2D

SCAN PLANE

42

24

26

Dα

# FIG.—2E

RADIAL PLANE

X-RAY BEAM

28

X—RAY BEAM EFFECTIVE FOCAL SPOT AXIAL WIDTH

ELECTRON BEAM

24

30

ELECTRON BEAM SPOT RADIAL WIDTH

TARGET 26

# FIG.—2F

SCAN PLANE

46

ELECTRON BEAM

X—RAY EFFECTIVE FOCAL SPOT SIZE

ELECTRON BEAM SPOT AZIMUTHAL WIDTH

30

# FIG.—2G

CYLINDRICAL SYMMETRY

$1/f_x - 1/f_y = 0$

900 mA

600 mA

300 mA

0 mA

RADIUS (cm)

z (cm)

**FIG.—3**

DATA 100 kV
350 mA
$1/f_y - 1/f_x = 0.16 m^{-1}$

y (cm) SCAN PLANE

z (cm)

x (cm) RADIAL PLANE

**FIG.—7**

$1/f_y - 1/f_x = 0.08 m^{-1}$

$x_0^1 - y_0^1 = 0.004$

100 kV

FIG.—4

FIG.—5

BEAM CURRENT OF 100 keV ELECTRONS

REDUCTION IN AXIAL DIMENSION DUE TO
TARGET ANGLE = 1/5

FIG.—6